# EUROPÄISCHE PATENTSCHRIFT

(11) **EP 0 536 598 B1**
(45) Veröffentlichungstag und Bekanntmachung des Hinweises auf die Patenterteilung: **26.11.1997**
(21) Anmeldenummer: 92116337.4
(22) Anmeldetag: 24.09.1992
(51) Int. Cl.: A61F 5/41

(54) **Prothese zur Verwendung bei Potenzstörungen**
Impotence prosthesis
Prothèse pour l'impuissance

(30) Priorität: 09.10.1991 DE 9112517 U
(43) Veröffentlichungstag der Anmeldung: 14.04.1993
(73) Patentinhaber: Dragunski, Heinz, 34454 Arolsen-Volkhardinghausen (DE)
(72) Erfinder: Dragunski, Heinz, 34454 Arolsen-Volkhardinghausen (DE)
(74) Vertreter: Herrmann-Trentepohl, Werner, Dipl.-Ing.

(56) Entgegenhaltungen:
- DE-A- 135 034
- FR-A- 2 012 026
- US-A- 2 868 192
- US-A- 3 455 301
- US-A- 4 240 413

## Beschreibung

Die Erfindung betrifft eine Prothese zur Verwendung bei Potenzstörungen.

Unter Potenzstörungen im Sinne der Erfindung werden Erektionsstörungen des Mannes, insbesondere Erektionsschwächen oder -unfähigkeit verstanden, die sowohl psychogene als auch symptomatische Ursachen, beispielsweise als Folge von Mißbildungen, Gefäßerkrankungen, endokrinen Störungen oder Operationen, haben können. Solche Fälle der Impotenz sind für den daran Erkrankten häufig mit großen seelischen Belastungen verbunden, so daß erhebliche Anstrengungen und Kosten zur Beseitigung bzw. Heilung unternommen werden.

Zur Behebung solcher Störungen werden häufig operative Eingriffe unternommen, die aber bei Mißlingen den zu behebenden Zustand häufig zementieren bzw. verschlimmern und daher nur in Notfällen angewandt werden. Hinzu kommt, daß bei derartigen Operationen die Gefahr weiterer Schädigung auftritt.

Es wurde auch eine Form der inneren Prothetik mittels in das männliche Glied eingepflanzter elastischer Implatate versucht, was sich aber für den Patienten häufig als unangenehm bzw. störend erwiesen hat.

US-A-2 868 192 beschreibt eine Prothese zur Anwendung bei Potenzstörungen mit den Merkmalen des Oberbegriffs des Anspruchs 1.

Es besteht daher ein Bedarf an einer einfachen, im Bedarfsfall schnell verfügbaren und wirksamen Prothese für Potenzstörungen, die äußerlich und ohne ärztliche Hilfe, insbesondere ohne operative Eingriffe anwendbar ist. Diese Aufgabe wird erfindungsgemäß mit einer Prothese gelöst, die als elastischer Hohlkörper mit einer Basisöffnung und einer oberen Öffnung ausgebildet ist, der sich von der im wesentlichen ovalen Basis zur im wesentlichen kreisförmigen oberen Öffnung gleichmäßig verjüngt, wobei die Achse durch das Zentrum beider Öffnungen in Richtung der großen Achse des Ovals gegen die Senkrechte gekippt ist, und der Hohlkörper die Form eines leicht gekippten Hohlkegels ohne Spitze aufweist, wobei die Wandung des Hohlkörpers entlang einwärts gekrümmter Linien verläuft.

Nach einer bevorzugten Ausführungsform besteht die Prothese aus einem handelsüblichen elastischen Silikonkautschuk, wobei die Steifigkeit des Materials auf das Ausmaß der Potenzstörungen abgestimmt ist. Zur Verstärkung der Prothesenwirkung kann dabei im Bereich des Übergangs von ovaler Basis zu kreisförmiger oberer Öffnung eine größere Wandstärke in Form eines an die Innenseite geringfügig vorstehenden ringförmigen Wulstes vorgesehen sein.

Zweckmäßigerweise verjüngt sich die Prothese von ihrer Basis zur oberen Öffnung entlang einwärts gekrümmter Linien so, daß eine stärkere Krümmung auf der Seite der Neigung gegenüber der Senkrechten vorgesehen ist.

Zweckmäßigerweise ist die Achse durch das Zentrum beider Öffnungen um etwa 5 bis 30° gegen die Senkrechte gekippt, insbesondere um etwa 10°.

Die Erfindung wird durch beiliegende Abbildungen, die sich auf eine bevorzugte Ausführungsform beziehen, näher erläutert. Von diesen Abbildungen zeigen
- Fig. 1: eine erfindungsgemäße Prothese in perspektivischer Ansicht und
- Fig. 2: einen Schnitt durch die Prothese gem. Fig. 1.

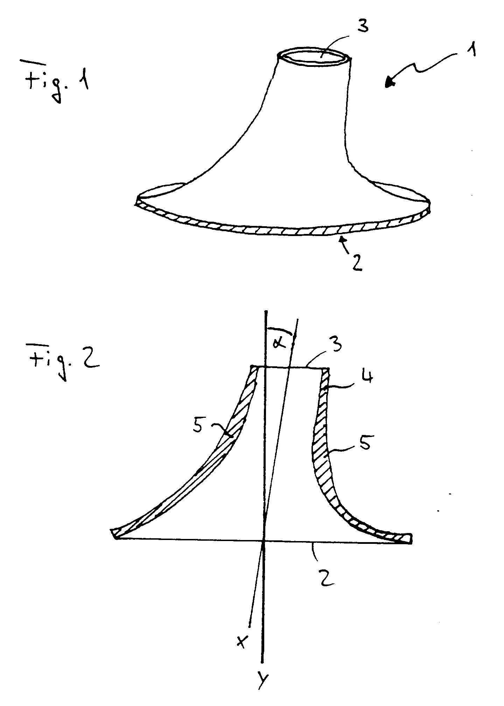

Fig. 1 zeigt in perspektivischer Ansicht eine bevorzugte Ausführungsform der erfindungsgemäßen Prothese in Form eines Hohlkörpers (1) mit einer offenen Basis (2) und einer Öffnung (3) an der Spitze. Der Hohlkörper selbst hat die Form eines leicht gekippten Hohlkegels ohne Spitze, dessen Wandung entlang einwärts gekrümmter Linien verläuft. Sie besteht vorzugsweise aus elastischem Silikonkautschuk und ist mit seinen Innenkonturen an die Wurzel und den unteren Schaft des männlichen Gliedes angepaßt.

Fig. 2 zeigt die Prothese gemäß Fig. 1 in seitlichem Schnitt, wobei eine Fertigung (5) der Wandung (4) im zentralen Bereich des Übergangs von Basis zu oberer Öffnung deutlich hervortritt. Die das Zentrum der oberen Öffnung (3) mit dem Zentrum der ovalen Öffnung (2) an der Basis verbindende Gerade (x) ist gegenüber der Senkrechten (y) im Zentrum der Basisöffnung (2) um einen Winkel α von etwa 5 bis 30°, vorzugsweise etwa 10° gekippt.

## Patentansprüche

1. Prothese zur Verwendung bei Potenzstörungen, die als elastischer Hohlkörper (1) mit einer Basisöffnung (2) und einer oberen Öffnung (3) ausgebildet ist, wobei sich der Hohlkörper (1) von der im wesentlichen ovalen Basis (2) zur im wesentlichen kreisförmigen oberen Öffnung (3) gleichmäßig verjüngt und die Achse (x) durch das Zentrum beider Öffnungen in Richtung der großen Achse des Ovals gegen die Senkrechte (y) gekippt ist und der Hohlkörper (1) die Form eines leicht gekippten Hohlkegels ohne Spitze aufweist, dadurch gekennzeichnet, daß die Wandung des Hohlkörpers (1) entlang einwärts gekrümmter Linien verläuft.

2. Prothese nach Anspruch 1, dadurch gekennzeichnet, daß sie aus Silikonkautschuk besteht.

3. Prothese nach Anspruch 1 oder 2, dadurch gekennzeichnet, daß die Wandung im Bereich des Übergangs zur oberen Öffnung verstärkt ausgebildet ist.

4. Prothese nach einem der Ansprüche 1 bis 3, dadurch gekennzeichnet, daß die Achse (x) durch das Zentrum beider Öffnungen um 5 bis 30° gegen die Senkrechte (y) gekippt ist.

## Claims

1. A prosthesis for use in cases of impotence which is constructed as a flexible hollow body (1) having a base aperture (2) and an upper aperture (3), whereby the hollow body (1) narrows conformably from the essentially oval base (2) to the essentially circular upper aperture (3) and the axis (x) through the centre of both apertures in the direction of the large axis of the oval section is inclined from the vertical line (y) and the hollow body (1) displays the shape of a gently inclined hollow cone without a top, characterised in that the wall of the hollow body (1) follows a course along inwardly curved lines.

2. A prosthesis according to claim 1, characterised in that it consists of silicone rubber.

3. A prosthesis according to claim 1 or 2, characterised in that the wall in the region of the transition to the upper aperture is strengthened.

4. A prosthesis according to one of the claims 1 to 3, characterised in that the axis (x) through the centre of both apertures is inclined by between 5° and 30° to the vertical line (y).

## Revendications

1. Prothèse à utiliser en cas de troubles provoqués par l'impuissance, qui est façonnée sous forme d'un corps creux élastique (1) comportant une ouverture à la base (2) et une ouverture supérieure (3), où le corps creux (1) se rétrécit uniformément depuis la base essentiellement ovale (2) jusqu'à l'ouverture supérieure essentiellement circulaire (3) et l'axe (x) qui passe par le centre des deux ouvertures est incliné par rapport à la verticale (y) en direction du grand axe de l'ovale et le corps creux (1) présente la forme d'un cône creux sans pointe légèrement incliné, caractérisée en ce que la paroi du corps creux (1) s'étend le long de lignes courbées vers l'intérieur.

2. Prothèse suivant la revendication 1, caractérisée en ce qu'elle se compose d'un caoutchouc de silicone.

3. Prothèse suivant la revendication 1 ou 2, caractérisée en ce que la paroi est renforcée dans la zone de la transition vers l'ouverture supérieure.

4. Prothèse suivant l'une quelconque des revendications 1 à 3, caractérisée en ce que l'axe (x) qui passe par le centre des deux ouvertures est incliné de 5 à 30° par rapport à la verticale (y).
